# EUROPEAN PATENT APPLICATION

(11) **EP 2 033 618 A1**
(43) Date of publication of application: **11.03.2009**
(21) Application number: 07115292.0
(22) Date of filing: 30.08.2007
(51) Int. Cl.: A61K 8/04, A61K 8/25, A61K 8/81, A61Q 5/06

(54) **Leave-in hair styling product with particles for improving hair volume**

(71) Applicant: Wella Aktiengesellschaft, 64274 Darmstadt (DE)
(72) Inventor: Birkel, Susanne, 64295 Darmstadt (DE); Schiemann, Hartmut, 36088 Hünfeld (DE)
(74) Representative: Schulze, Rainer

(57) **Abstract**

The present invention relates to a leave-in hair styling product that improves the volume of a hair-do. More specifically, it relates to a leave-in composition in the form of aerosol mousse, pump mousse, gel, spray-gel, aerosol hairspray, pump spray, hair wax, hair styling cream or hair setting lotion. The leave-in compositions according to the present invention comprise solid particles of spherical, ellipsoid or oval shape and at least one film-forming ingredient.

## Description

### FIELD OF THE INVENTION

The present invention relates to a leave-in hair styling product that improves the volume of a hair-do. More specifically, it relates to a leave-in hair styling product such as aerosol mousse, pump mousse, gel, spray-gel, aerosol hairspray, pump spray, hair wax, hair styling cream or hair setting lotion.

### BACKGROUND OF THE INVENTION

It's common to hear someone complain about a "bad hair day." "Bad hair", depending on the owner, can be too flat, too frizzy, or simply uncontrollable. "Flat hair" is usually the complaint of people who have fine, thin hair. In order to achieve good hair volume, which is the visible bulkiness of hair; these people desire more body and fullness from their hair. There are many factors that influence hair body and fullness: hair diameter, hair fiber-to-fiber interactions, natural configuration (kinky, straight, wavy), bending stiffness, hair density (number per cm²), and hair length.

In an attempt to increase hair volume, people have used styling products to alter fiber-to-fiber interactions and lock their created styles in place. This is particularly true for the hair styling gels and mousse products. During their wet state on hair, gel and mousse products help in increasing the grab of hair fibers by a hair comb or brush and thereby help in the creation of a hair style. On drying, polymeric bonds are formed in between and on the surface of hair fibers.

These bonds help in holding and maintaining the created hair volume and style. In addition, polymeric bonds formed by styling products are broken when the hair is combed or brushed.

Broken bonds have jagged edges which enhance inter-fiber friction and hence help in maintaining the hair volume and style. However, styling products such as these can leave the hair feeling stiff, tacky and/or sticky.

Other techniques used to increase the appearance of hair volume include perming, hair straightening, back combing, and pressing. These processes are all focused on trying to change the nature of a person's hair substrate. However, all of these processes can damage the hair.

Other attempts to increase the diameter of the hair have resulted in insignificant gains or in severe hair damage. Therefore, a need still exists for a means to improve hair volume while maintaining good hair feel. Especially, there is a need for compositions which provide increased volume which is also long-lasting under increased humidity and ideally is long lasting to the effect that it survives to a noticeable degree at least one or more hair washings.

### SUMMARY OF THE INVENTION

The present invention is directed to a leave-in hair styling product for improving the appearance of volume of a hair-do. The product is in the form of an aerosol mousse, pump mousse, gel, spray-gel, aerosol hairspray, pump spray, hair wax, hair styling cream or hair setting lotion and the product comprises a composition, said composition comprising a liquid carrier, at least one hair volume increasing, solid particulate materials and at least one film-forming ingredient. The solid particulate materials are solid particles of spherical, ellipsoid or oval shape having a mean particle size of from 5 µm to 80 µm, a water content of below 5 wt.% and a melting point of at least 90°C. The solid particulate materials also can be a mixture of at least one first solid material and at least one second solid material wherein said first solid material is at least one irregularly shaped particle and said second solid material is at least one spherical particle, wherein said irregularly shaped particles have a particle size of at least 0,01 micron, said spherical shaped particles have a particle size of at least 0,01 micron and the median particle size of said spherical particle is greater than the median particle size of said irregular shaped particle. In a preferred embodiment, the total amount of liquid carrier is from 20 to about 95 wt.% (preferably from 55 to 85 wt.%), the total amount of particles is from 0,005 to 5 wt.% and the total amount of film-forming polymers is from 0,1 to 15 wt.% (preferably from 0,5 to 10 wt.%).

The present invention is further directed to a method of treating hair and to the use of the leave-in hair styling product for improving the volume of a hair-do.

These and other features, aspects, and advantages of the present invention will become evident to those skilled in the art from a reading of the present disclosure.

### BRIEF DESCRIPTION OF THE DRAWINGS

**Figure 1**: SEM photo of Microthene FN 510-00 particles,
**Figure 2****:** SEM photo of Sipemat 22 LS particles,
Figure 3: SEM photo of hair tress treated with volume mousse of Example 1,

### DETAILED DESCRIPTION OF THE INVENTION

While the specification concludes with claims which particularly point out and distinctly claim the invention, it is believed the present invention will be better understood from the following description.

The leave-in compositions of the present invention include a liquid carrier, and specific solid particulate materials and at least one film-forming ingredient. Each of these essential components, as well as preferred or optional components, is described in detail hereinafter.

All percentages, parts and ratios are based upon the total weight of the compositions of the present invention, unless otherwise specified.

Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

The term "fluid" as used herein, means a liquid or a gas which tends to take the shape of its container, container being the wall of the flexible hollow particles. The term "solid" as used herein, means a non-fluid and non-waxy material.

The term "irregular" as used herein, means a particle that has a non-uniform surface texture and/or no spherical, oval, ellipsoid or other uniform shape. The term "spherical" as used herein, means a spherical body which is the set of points in a metric space whose distance from a fixed point is approximately constant. Here, the meaning of "approximately" is that the fixed points are within a distance of ±15%.

The term "hollow" as used herein, means a particle having an encapsulated area that is substantially free of solid mass, the encapsulated area comprising from 10 to 99.8 percent of the total volume of the particle. The term "fluid-encapsulated" as used herein, means that the hollow particles of the invention are structurally hollow. In accordance with the invention, the term "structurally hollow" nonetheless allows the hollow particles to contain at least one additional fluid material therein.

The term "polymer" as used herein shall include materials whether made by polymerization of one type of monomer (i.e. homopolymers) or made by two or more types of monomers (i.e. copolymers).

The term "particle size" as used herein means the diameter of the particle. For non-spherical particles, the particle size refers to the smallest diameter of the particles, i.e. the diameter in the dimension having the smallest diameter.

The term "suitable for application to human hair" as used herein, means that the compositions or components thereof so described are suitable for use in contact with human hair and the scalp and skin without undue toxicity, incompatibility, instability, allergic response, and the like.

The term "water soluble" as used herein, means that a material is soluble in water in the present composition. In general, the material should be soluble at 25° C at a concentration of 1%, more preferably at 5%, most preferably at 15%. The term "water insoluble" as used herein, means that a material is insoluble in water in the present composition. In general, the material should be soluble at 25° C at a concentration of less than 1% by weight of the water solvent.

The term "leave-in composition" as used herein refers to compositions which are applied to the human hair and which are not washed out immediately thereafter or after a relatively short period, such as less than 1 hour (as is typically the case for compositions used to shampoo or to condition the hair).

The term "film-forming polymers" as used herein is defined as those polymers, which are in a position to deposit a polymer film on the hair when used in a concentration of 0.01 to 5 % by weight aqueous, alcoholic or aqueous-alcoholic is solution.

### Liquid Carrier

The compositions of the present invention comprise a liquid carrier, which is preferably present at a level of from about 20% to about 95%, preferably from about 55% to about 85%, by weight of the compositions.

The liquid carrier can be either a purely aqueous medium or an aqueous-alcoholic medium with preferably up to 40 percent by weight alcohol. Lower univalent or multivalent alcohols suitable for cosmetic purposes and having from one to five carbon atoms, such as, e.g., ethanol, isopropanol, n-propanol, ethylene glycol, glycerol and propylene glycols, especially 1,2-propylene glycol, may be used as the alcohol. Typical water content is from 55 to 95 percent by weight and preferably from 65 to 80 percent by weight. Typical alcohol content is from 0 to 30 percent by weight and preferably from 1 to 25 percent by weight. Preferably, the liquid carrier is selected from water, ethanol and isopropanol and mixtures thereof.

### Particles of spherical, oval or ellipsoid shape

The composition of the present invention includes particles. The particles are water insoluble solid, non-waxy particles and have a spherical, oval or ellipsoid shape having a mean particle size of from 5 µm to 80 µm, a water content of below 5 wt.% and the solid particals do not melt at temperatures below 90°C, preferably not below 160°C. For ellipsoid or oval shaped particles the ratio of the largest dimension to the smallest dimension (defined as the aspect ratio) is less than 10, preferably is less than 8 and even more preferably is less than 5.

The particle may be colored or non-colored (for example transparent). However, the particles are not suitable to impart a color-effect to the hair. Thus, if colored particles are used, they preferably match the color of the hair.

Preferably the particles comprise at least one polyolefin such as polyethylene or polypropylene. More preferably the material, of which the particles are made, comprises at least 50% of a polyolefin, more preferably at least 70%, at least 80% and even more preferably at least 90%. In the most preferred embodiment the material of which the particles are made consists only of polyolefin. Preferred polyolefins are nylon, polyethylene, polyester, polypropylene, polystyrene, polytetrafluoroethylene, polyurethane, polyamide, epoxy resins, urea resins, and mixtures thereof, with the most preferred polyolefins being polyethylene and polypropylene.

Non limiting examples of useful particles are Microease INS, INS, 116 (micronized synthetic waxes), Micropoly 210, 250S (micronized polyethylene), Microslip (micronized polytetrafluoroethylene), and Microsilk (combination of polyethylene and polytetrafluoroethylene), all of which are available from Micro Powder, Inc. Other examples include MP-2200, BPA-500 (polymethylmethacrylate), EA- 209 (ethylene/acrylate copolymer), SP-501(nylon-12), SP-10 (nylon-12), ES- 830 (polymethyl methacrylate), BPD-800, BPD-500, BPA-500 (polyurethane) and CL2080 (polyethylene) particles available from Kobo Products, Inc., polyethylene powders such as those available from Quantum Chemical under the trade name Microthene including MN701, MN710, MN-714, MN-722 and FN5100 and those available from Shamrock Technologies, Inc. under the trade name CeraPURE, nylon particles such as those available from available from Elf Atochem under the trade name Orgasol, acrylates copolymers available from Advanced Polymer Systems under the trade name Microsponge and Polytrap, polytetrafluoroethylene powders such as those available from Shamrock Technologies, Inc. under the tradenames FluoroPURE and HydroPURE, and silicone resins sold under the name Tospearl particles by GE Silicones including Tospearl 105, 120, 130, 145, 3120 and 240. Ganzpearl GS-0605 crosslinked polystyrene (available from Presperse) is also useful.

Non limiting examples of hybrid particles include Ganzpearl GSC- 30SR (Sericite & crosslinked polystyrene hybrid powder), SM-1000, SM- 200 (mica and silica hybrid powder available from Presperse), and FluroTOUCH 135C and 235C (polyethylene and polytetrafluoroethylene available from Shamrock Technologies, Inc).

Especially preferred particles of the present invention are Microthene FN-510-00 available from Equistar Chem., Houston, USA and Cerapure H-540-C available from Shamrock Technologies, Inc., USA.

Suitable powders include bismuth oxychloride, titanated mica, polymethylmethacrylate, micronized teflon, acrylate polymers, aluminum starch octenylsuccinate, chalk, corn starch, glyceryl starch, magnesium oxide, chitin, microcrystalline wax, polyethylene, polypropylene, polyethylene terephtalate, polyacrylate, cellulose, solid keratin particles, magnesium silicate, maltodextrin, rice starch, talc, mica, titanium dioxide, zinc laurate, zinc myristate, zinc neodecanoate, zinc resinate, zinc stearate, alumina, attapulgite, calcium carbonate, calcium silicate, dextran, nylon, perlon, silica silylate, silk, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature.

The particles of the present invention can have surface charges or their surface can be modified with organic or inorganic materials such as surfactants, polymers, and inorganic materials. The surface of the particle may be charged through a static development or with the attachment of various ionic groups directly or linked via short, long or branched alkyl groups. However, in a preferred embodiment the particles are neutral, i.e. they do not have any positive or negative charges.

In one embodiment of the present invention, the particles used in the composition are hollow particles. In a preferred embodiment, the hollow particles are fluid-encapsulated, flexible microspheres. The microspheres are structurally hollow, however, they may contain various fluids, i.e. liquids and gases and their isomers. Suitable liquids may contain vitamins, amino acids, proteins and protein derivatives, herbal extracts, pigments, dyes, antimicrobial agents, chelating agents, UV absorbers, optical brighteners, silicone compounds, perfumes, humectants, conditioning agents, and mixtures thereof.

The particles of the present invention are solid. They should also be form stable, i.e. they maintain their structural integrity during normal use of the composition. More preferably, at least 90% all of the particles in the composition maintain their structural integrity during normal use of the composition.

Preferred particles will also have physical properties which are not significantly affected by typical processing of the composition. The particles have melting points greater than 90°C, more preferably greater than 95°C and even more preferably more than 100°C or at least 160°C. Preferably, the particles do not melt once applied onto the hair, even if the hair is dried with a hair blower. Thus, waxes are not suitable for use as particles in the present invention.

Also, the particles have a water content of below 5% by weight of the particles. Thus, gel particles are not suitable for the present invention as they are typically too soft. If the water content is above 5% by weight of the particles, the particles may dry out after being applied onto the hair. However, upon drying, the may shrink, thereby reducing the volume effect, they achieve in the hair. To ensure that the water content does not increase, the particles should also not swell, i.e. they should not absorb water.

Further, the particles preferably have a density of less than 2.1 g/cm³, more preferably of less than 1.9 g/cm³ and even more preferably of less than 1.6 g/cm³. If the density of the particles is higher than 2.1 g/cm³, the particles may add too much weight to the hair, thus reducing the volume effect. This applies especially to relatively large particles and for compositions with relatively high particle concentrations. For example, particles consisting of silicumdioxide, would have a relatively high density (SiO₂ has a density from 2.19 to 2.66 g/cm³; Römpps Chemie Lexikon, 8. Auflage 1997).

The particles of the present invention have a mean particle size of from 5 µm to 80 µm. Preferable, the particles have a mean particle size of from 10 µm to 60 µm, even more preferably from 10 µm to 50 µm. If the mean particle size is below 5 µm, the desired volume effect may not be achieved. On the other hand, if the mean particle size is above 80 µm, the particles may become too large and thus may become visible with the naked eye. Also, the particles may become too heavy, thereby deteriorate the volume-effect.

Preferably, at least 80% of the spherical, oval or ellipsoid shaped particles comprised in the leave-in composition of the present invention have a mean particle size of from 5 µm to 80 µm. More preferably at least 80% of the spherical, oval or ellipsoid shaped particles comprised in the composition have a mean particle size of from 10 µm to 60 µm, and even more preferably at least 80% of the spherical, oval or ellipsoid shaped particles comprised in the composition have a mean particle size of from 10 µm to 50 µm.

Still more preferably, at least 90% of the of the spherical, oval or ellipsoid shaped particles comprised in the leave-in composition of the present invention have a mean particle size of from 5 µm to 80 µm. More preferably at least 90% of the spherical, oval or ellipsoid shaped particles comprised in the composition have a mean particle size of from 10 µm to 60 µm, and even more preferably at least 90% of the spherical, oval or ellipsoid shaped particles comprised in the composition have a mean particle size of from 10 µm to 50 µm.

The compositions of the present invention should comprise at least 0.005 wt.% of particles having a mean particle size of from 5 µm to 80 µm, more preferably at least 0.01 wt% and still more preferred at least 0.5 wt%. Further, the compositions of the present invention do not have to comprise more than 8 wt%, more preferably not more than 5 wt% and still more preferable not more than 3 wt%.

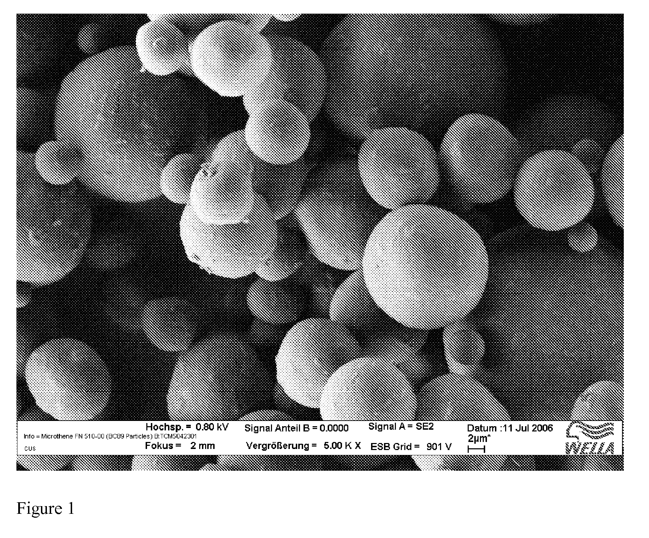

The particles of the present invention should have a smooth surface, i.e. the surface (e.g. if viewed with SEM) should appear smooth. E.g. for spherical particles having a smooth surface, the spherical particle is defined by a set of points in a metric space whose distance from a fixed point is approximately constant. Here, the meaning of "approximately" is that the fixed points are within a distance of ±15%, preferably of ± 10%, even more preferably of ± 5%. A similar surface morphology preferably also applies for ellipsoid particles. Figure 1 shows a SEM photo of Microthene FN 510-00 particles as an example for spherical particles according to the present invention. The SEM used is a Zeiss Ultra 55.

### Film-forming ingredients

Preferred film-forming ingredients are film-forming polymers. Suitable natural film-forming polymers with hair-fixing action include chitosan. The chitosan may be of low molecular weight Chitosan having a molecular weight of 30,000 to 70,000 g/mol that is sold for example by Kyowa Oil & Fat Co., Japan or the chitosan can be of high molecular weight, e.g. 200,000 and more. Different saccharide types can be used such as polysaccharides or mixtures of oligo-, mono- and disaccharides, which are sold for example under the trademark C-PUR^{®} by Cerestar, Brussels. Also suitable natural polymers include Chinese Balsam resin and cellulose derivatives, e.g. hydroxypropyl cellulose with a molecular weight of 30,000 to 50,000 g/mol, that is sold for example under the trademark Nisso S1^{®} by Lehmann & Voss, Hamburg. Shellac, such as sold under the tradename shellac MNP 210 by Fa. Pennig, Hamburg, is an additional suitable natural polymer. Shellac can be used in neutralized and unneutralized form.

Further examples of neutral film-forming polymers suitable in the present invention are hydroxypropylcellulose, e.g. sold under the tradename Klucel^{®} by Hercules Incorporated, derivatives of chitosan, e.g. N-hydroxypropylchitosanisopropylether as described in EP 277 322, N-hydroxyalkylchitosanbenzylether as described in EP 300 234 and alkyl-hydroxypropyl-substituted chitosan derivatives as described in EP 193 736; polylactide/polylactid-copolymers as described in DE 100 27 393; polycrotonbetain-oligomers and -polymers as described in DE 10027391.

Suitable synthetic film-forming anionic polymers include, e.g., crotonic acid/vinyl acetate copolymers, which for example are sold in the form of a 60% solution in isopropanol/water under the trademark Aristoflex^{®} by Hoechst. Additional suitable anionic polymers are, e.g. terpolymers of acrylic acid, ethyl acrylate and N-t-butylacrylamide, such as those marketed under the trademark Ultrahold^{®} 8 and Ultrahold^{®} strong of BASF.

Further suitable anionic polymers according to the present invention are salts of polystyrenesulfonic acid, the sodium salts having a weight average molecular weight ranging from about 100,000 to about 500,000 sold under the tradename Flexan^{®} 500 and Flexan^{®} 130 by National Starch; copolymers of t-butyl acrylate, ethyl acrylate, methacrylic acid (e.g. Luvimer^{®} 100P); VA/Crotonates/Vinyl Neodecanoate Copolymer sold under the tradename Resyn^{®} 28-2930 by National Starch & Chemical Company; VA/Crotonates Copolymer sold under the tradename Luviset^{®} CA66 by BASF; Butyl Ester of PVM/MA Copolymer sold under the tradename Gantrez^{®} ES-425 by International Speciality Products (ISP); AMP-Acrylates/Allyl Methacrylate Copolymer sold under the tradename Fixate^{®} G-100 by Noveon Inc.; Polyacrylate-14 sold under the tradename Fixate^{®} PLUS by Noveon Inc.; Ethyl Ester of PVM/MA Copolymer sold under the tradename Omnirez^{®} 2000 by ISP.

According to one embodiment of the invention, preferred film-forming ingredients are cationic film-forming polymers. Preferred cationic polymers that can be used in the compositions of the invention include, e.g., polymers comprising monomeric units of quaternized dialkyl aminoalkylmethacrylate and polymers comprising monomeric units of vinylimidazolin. The alkyl groups may comprise from 1 to 4 C-atoms. An example of a preferred cationic polymer is quatemized polyvinyl pyrrolidone/ dimethyl aminoethylmethacrylate polymer (polyquatemium-11), that are for example sold under the trademark Gafquat^{®} 755 N by ISP and is the polymeric salt formed by the reaction of diethyl sulfate and a copolymer of vinyl pyrrolidone and dimethyl aminoethylmethacrylate. Another example of a preferred cationic polymer is the copolymer of N-vinylpyrrolidone, N-vinylimidazol, methacrylamide and quatemized N-vinylimidazol, such as polyquatemium-68 which is the polymeric quaternary ammonium salt formed by the polymerization of vinylpyrrolidone, methacrylamide, vinylimidazole and 3-methyl-1-vinylimidazolium methylsulfate and is provided as Luviquat Supreme by BASF.

Additional cationic polymers include, for example, copolymers of polyvinyl pyrrolidone and imidazolimine methochloride, such as those sold under the trademark Luviquat^{®} HM 550 by BASF; the terpolymer of dimethyldiallyl ammonium chloride, sodium acrylate and acrylamide sold under the trademark Merquat^{®} Plus 3300 by Calgon Pittsburgh, PA, U.S.A.; the terpolymer of vinyl pyrrolidone, dimethyl aminoethyl methacrylate and vinyl caprolactam that is marketed by ISP Co, U.S.A. under the trademark Gaffix^{®} VC 713; the quaternary ammonium salt of hydroxyethyl cellulose and a trimethylammonium substituted epoxide sold under the trademark Polymer IR^{®} by Amerchol, Edison, N.J., U.S.A.; the vinylpyrrolidone/ methacrylamidopropyl trimethylammonium chloride copolymer (polyquatemium-28) sold under the trademark Gafquat^{®} HS 100 of ISP and diquatemary polydimethylsiloxane (quatemium-80) sold under the trademark Abil^{®} Quat 3272 by Degussa Care & Surface Specialities; polyquatemium-4 (PQ-4) sold under the tradename Celquat^{®} H-100 by National Starch; polyquatemium-10 (PQ-10) sold under the tradename Celquat^{®} SC-2040C by National Starch; polyquatemium-6 (PQ-6) sold under the tradename Merquat^{®} 100 by Nalco Company; polyquaternium-7 (PQ-7) sold under the tradename Merquat^{®} 2200 by Nalco Company; polyquaternium-16 (PQ-16) sold under the tradename Luviquat^{®} FC 370 by BASF; polyquatemium-35 (PQ-35) sold under the tradename Plex^{®} 3074 L by Röhm GmbH Chemische Fabrik; polyquatemium-37 (PQ-37) sold under the tradename Salcare^{®} SC95 by Ciba Speciality Chemicals Corp.; polyquatemium-57 (PQ-57) sold under the tradename Zeniberry^{®} C-XC by Zenitech LLC; polyquatemium-47 (PQ-47) sold under the tradename Merquat^{®} 2001 by Nalco Company.

Suitable amphoteric polymers include, e.g., copolymers of octoylacrylamide, t-butylamino-ethylmethacrylate and two or more monomers, comprising acrylic acid, methacrylic acid or their esters, such as are obtainable from National Starch, U.S.A under the trademark Resyn^{®} 28-4910 or Amphomer^{®} LV-71.

Further amphoteric polymers suitable in the present invention include isobutylene/ ethylmaleimide/hydroxyethylmaleimide copolymer sold under the tradename Aquaflex^{®} FX 64 available from ISP.

Suitable synthetic nonionic film-forming, hair-fixing polymers include homopolymers of vinyl pyrrolidones, which for example are sold under the trademark, Luviskol^{®} K of BASF or PVP-K of ISP, Wayne, N.J., U.S.A. and homopolymers of N-vinyl formamides, such as those sold under the tradename PVF of National Starch. Furthermore suitable synthetic film-forming, nonionic hair-fixing polymers include, e.g., copolymerizates of vinyl pyrrolidone and vinyl acetate that are sold under the trademark Luviskol^{®} VA of BASF; terpolymers of vinyl pyrrolidone, vinyl acetate and vinyl propionate, that are sold, for example, under the trademark, Luviskol^{®} VAP of BASF; polyacrylamides, such as those sold under the trademarks Akyponines^{®} 191 of CHEM-Y, Emmerich or Sepigel^{®} 305 of Seppic Co; polyvinyl alcohols, which are sold under the trademarks Elvanol^{®} of Du Pont, or Vinol^{®} 523/540 of Air Products and polyethyleneglycol/polypropylene glycol copolymers, that are sold under the trademark Ucon^{®} of Union Carbide; VP/vinyl caprolactam/DMAPA acrylat copolymer sold under the tradename Aquafle^{®} SF-40 by ISP.

Preferred film-forming ingredients are selected from the group consisting of cationic polymers and nonionic vinyllactam polymers and mixtures thereof. Preferred cationic polymers are selected from the group consisting of polymers comprising monomeric units of quaternized dialkyl aminoalkylmethacrylate and polymers comprising monomeric units of quatemized vinylimidazolin. Preferred nonic vinyllactam polymers are selected from polyvinylpyrrolidone and copolymers of vinylpyrrolidone and vinylacetate and mixtures thereof. Most preferred cationic polymers are selected from the group consisting of copolymers ofN-vinylpyrrolidone, N-vinylimidazol, methacrylamide and quatemized N-vinylimidazol, copolymers of vinyl pyrrolidone and quatemized dimethyl aminoethylmethacrylate and copolymers of N-vinylpyrrolidone and quatemized N-vinylimidazol and mixtures thereof.

Furthermore polymers with a thickening action can additionally be used if they are compatible with the remaining ingredients of the compositions according to the invention.

Homopolymers of acrylic acid with a molecular weight of from 2,000,000 to 6,000,000, such as those sold by BF Goodrich, Cleveland, Ohio under the trademark Carbopol^{®} can be used as thickeners in the compositions according to the invention. As an additional thickener acrylic acid homopolymers with a molecular weight of 4,000,000, such as those sold by Goodrich under the trademark Carbopol^{®} 940 can be used in the compositions according to the invention. Additional thickeners include for example acrylic acid homopolymers sold under the tradename Modarez V 600 PX by Protex, France or by Goodrich under the trademark Carbopol^{®} ETD 2001; polymers of acrylic acid and acrylamide (sodium salts with a molecular weight of 2,000,000 to 6,000,000) sold by Hoechst under the trademark Hostacerin^{®} PN 73 and Sclerotium Gum sold by Alban Muller Montreal under the trademark Amigel^{®}. The copolymers of acrylic acid or methacrylic acid, such as those sold by Goodrich under the trademark Carbopol^{®} 1342 or Pemulen^{®} TR1 are particularly preferred.

Without being bound by theory, it is believed that the film-forming ingredient attaches to the hair and the spherical, oval or ellipsoid particles of the present invention are bound to the hair via the film-forming ingredient. Due to the size of the particles, neighbouring hairs are not more able to lie close to each other, as the particles form a "bridge" between the hairs, thus increasing the volume of the hair. Also, if hairs cross each other, the hairs are linked to each other via bridges of film-forming ingredients and particles, thereby further increasing hair volume. As the particles are spherical, oval or ellipsoid and not irregularly shaped, the particles impart a smooth touch to the hair. Contrary thereto, compositions comprising no such particles tend to impart a rough feeling to the hair. The smooth touch is even improved if the particles have a smooth surface.

### Small, irregular shaped particles

The composition according to the present invention may additionally comprise irregularly shaped particles. In one embodiment of the invention, the composition comprises a mixture of at least one first solid material and at least one second solid material wherein said first solid material is at least one irregularly shaped particle and said second solid material is at least one spherical particle, wherein said irregularly shaped particles have a particle size of at least 0,01 micron, said spherical shaped particles have a particle size of at least 0,01 micron and the median particle size of said spherical particle is greater than the median particle size of said irregular shaped particle. In one embodiment of the present invention, the weight ratio of spherical particles to irregularly shaped particles in the composition is at least about 1:1, preferably at least about 1.5:1, still more preferably at least about 2:1, and still more preferably at least about 2.5:1.

Irregularly shaped particles are particles which lack a uniform shape. Irregularly shaped particles are typically obtained through precipitation, grinding or pulverizing, or are comprised of fused or aggregated primary particles to yield particles with irregular shape or surface texture. The irregularly shaped particles of the present invention preferably have a particle size of less than 30 µm Typically, the particles will have a particle size from about 0.01 µm to about 20 µm, still more preferably from about 0.1 µm to about 15 µm, and even more preferably from about 0.5 µm to about 9 µm in diameter.

In the compositions of the present invention, it is preferable to incorporate at least 0.005% by weight of irregularly shaped particles, more preferably at least 0.01 %, still more preferably at least 0.05% by weight of particles. In the compositions of the present invention, it is preferable to incorporate no more than about 10% by weight of particles, more preferably no more than about 5%, still more preferably no more than 3%, and even more preferably no more than 2% by weight of irregularly shaped particles. To improve hair volume, the particles should preferably be able of conferring friction to reduce disruption and collapse of the hair style. Determination of the levels and particle types is within the skill of the artisan. Particles that are generally recognized as safe, and are listed in C.T.F. A. Cosmetic Ingredient Handbook, Sixth Ed., Cosmetic and Fragrance Assn., Inc., Washington D.C. (11th Edition, 2006), incorporated herein by reference, can be used.

The particle may be colored or non-colored (for example, white). Suitable irregularly shaped particles include for example fumed silica, polymethylmethacrylate, micronized teflon, boron nitride, barium sulfate, acrylate polymers, aluminum silicate, aluminum starch octenylsuccinate, calcium silicate, cellulose, chalk, corn starch, diatomaceous earth, fuller's earth, glyceryl starch, hydrated silica, magnesium carbonate, magnesium hydroxide, magnesium oxide, magnesium trisilicate, maltodextrin, microcrystaline cellulose, rice starch, silica, titanium dioxide, zinc laurate, zinc myristate, zinc neodecanoate, zinc resinate, zinc stearate, polyethylene, alumina, attapulgite, calcium carbonate, calcium silicate, dextran, nylon, silica silylate, silk powder, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature. The irregularly shaped particle component may also comprise various organic and inorganic pigments. The organic pigments are generally various aromatic types including ado, indigoid, triphenylmethane, anthraquinone, and xanthine dyes which are designated as D&C and FD&C blues, browns, greens, oranges, reds, yellows, etc. Organic pigments generally consist of insoluble metallic salts of certified color additives, referred to as the Lakes. Inorganic pigments include iron oxides, ultramarine and chromium or chromium hydroxide colors, and mixtures thereof.

Water insoluble irregularly shaped particles of various shapes and densities are useful. Preferred irregularly shaped particles tend to have a shape in which the ratio of the largest dimension to the smallest dimension (deemed as the aspect ratio) is less than 10. More preferably, the aspect ratio of the particles is less than 8. Still more preferably, the aspect ratio of the particles is less than 5. Particles useful in the present invention can be inorganic, synthetic, or semi- synthetic in composition. Hybrid particles are also useful. Synthetic particles can be made of either cross-linked or non cross-linked polymers. The particles of the present invention can have surface charges or their surface can be modified with organic or inorganic materials such as surfactants, polymers, and inorganic materials. Particle complexes are also useful.

Examples of useful inorganic particles include various silica particles including colloidal silicas, filmed silicas, precipitated silicas and silica gels. Non-limiting examples of colloidal silicas include Snowtex C, Snowtex O. Snowtex 50, Snowtex OL, Snowtex ZL available from Nissan Chemical America Corporation and colloidal silicas sold under the tradename Ludox available from W.R. Grace & Co. Non- limiting examples of filmed silicas include hydrophillic and hydrophobic forms available as Aerosil 130, Aerosil 200, Aerosil 300, Aerosil R972 and Aerosil R812 available from Degussa Corp. and those available from Cabot Corp. under the trade name Cab-O-Sil including Cab-O-Sil M-5, MS-5, TS-530, TS-610, and TS-720. Non-limiting examples of precipitated silicas include those available in both hydrophillic and hydrophobic versions from Degussa Corp. under the trade name Sipenat including Sipemat 350, 360, 22LS, I 22S, 320, 50S, D10, D11, D17, and C630; those sold by W. R. Grace & Co. under the trade name Syloid, those sold by the J.M. Huber Corp. under the tradename Zeothix and Zeodent, and those available from Rhodia under the trade name Tixosil. Other non-limiting examples of useful inorganic particles include various metallic oxides including titanium dioxide such as P25 available from Degussa, Tronox CR-840 available from Kerr McGee Chemical Corp, and MT 500B and MT-TOOT available from Tayca Corp. and aluminum oxide such as Aluminum Oxide C available from Degussa Corp and AC720, AC 712, and AC740 from AluChem Inc. Other useful i inorganic particles include silicate glass particles such as Glamur Glo Glass Chips available from Nippon Paint Corp. Examples of useful organic irregularly shaped particles include synthetic resin particles with irregular surface textures and non-spherical shape. Non-limiting examples include silicone resin particles available as Tospearl 240 and SR1000 available from GE Silicones.

Preferred particles will also have physical properties that are not significantly affected by typical processing of the composition. Preferably, particles having melting points greater than about 70°C are used. Still more preferably, particles having a melting point greater than 80°C are used and most preferably particles having melting point of greater than about 95°C or 160°C are used. As used herein, melting point would refer to the temperature at which the particle transitions to a liquid or fluid state or undergoes significant deformation or physical property changes. In addition, many of the particles of present invention are cross-linked or have a cross-linked surface membrane. These particles do not exhibit a distinct melting point. Cross-linked particles are also useful as long as they are stable under the processing and storage conditions used in the making of I the present compositions. Preferably, the particles are not drug actives. More preferably, the particles are not anti-dandruff actives.

Preferred irregularly shaped particles include hydrophilic and hydrophobically modified precipitated silicas and aluminas. Particularly preferred irregularly shaped particles include hydrophilic and hydrophobically modified precipitated silicas.

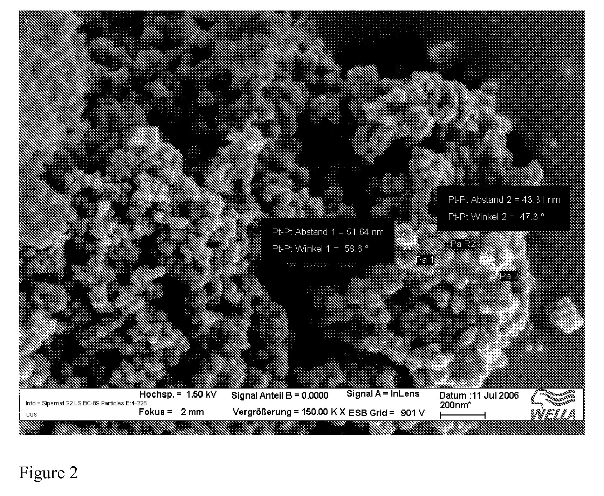

Figure 2 shows a SEM photo of Sipemat 22 LS particles as an example for small, irregular shaped particles according to the present invention. The SEM used is a Zeiss Ultra 55.

### Platelet particles

The composition of the present invention may further include platelet particles, provided they are physically and chemically compatible with the essential components described herein, or do not otherwise unduly impair product stability, aesthetics or performance. Platelet particles according to the invention are particles in which the ratio of the largest dimension to the smallest dimension (deemed as the aspect ratio) is greater than about 10. Platelet particles are typically used in cleansing compositions to impart desired aesthetic benefits to the composition, such as color or pearlescence, and do not typically deliver the desired hair friction or volume, fullness and style benefits desired. However, it has been found that particles with an aspect ratio of greater than 10 may be useful if they remain as aggregated particle stacks in the composition.

Non-limiting examples of platelet particles include various natural and synthetic silicate materials including talc, mica, sericite, titanated micas, magnesium aluminum silicates, aluminum silicate, calcium silicate, clays, bentonite, hectorite, montmorillonite. Other non-limiting examples of platelet materials include bismuth oxychloride, boron nitride, and platelet titanium dioxides. Platelet particles of the present invention can have surface charges or their surface can be modified with organic or inorganic materials such as surfactants, polymers, and inorganic materials. Non-limiting examples of commercially available platelet particles include Laponite XLS, Laponite SCPX-2549, Claytone SO and Gelwhite H NF from Southern Clay Products Inc.; Bentone 38, Bentone 27, and Bentone 34 available from Rheox, Inc.; Flamenco Ultra Silk 2500, Flamenco Satin Pearl 3500 and Timica Silkwhite 110W from Engelhard Corp; magnesium aluminum silicates marketed under the tradename Veegum available from R.T. Vanderbilt Company, Inc.; and Ultra Talc 2000, 3000, and 5000 available from Ultra Chemical.

### Additional Components

The compositions of the present invention may further comprise one or more optional components known for use in hair care or personal care products, provided that the optional components are physically and chemically compatible with the essential components described herein, or do not otherwise unduly impair product stability, aesthetics or performance. Individual concentrations of such optional components may range from about 0.001 % to about 10% by weight of the compositions.

The compositions according to the invention can also contain conventional additive ingredients suitable for hair treatment compositions. These additive ingredients include, e.g. moisturizers, viscosity modifiers such as thickener, pearlescent aids, additional surfactants or nonionic co-surfactants, pH adjusting agents, preservatives, chelants, proteins, sunscreens, UV absorbers, natural and/or synthetic oils and waxes (e.g. Candelillawax, Olive wax, camaubawax, paraffine waxes) and vitamins. Also, the compositions may comprise perfume oils in an amount of 0.1 to 0.5 percent by weight; bactericidal and fungicidal agents, for example, 2,4,4 trichloro-2-hydroxydiphenyl ether or methyl chloroisothiazolione, in an amount of from 0.01 to 1.0 percent by weight; buffer substances, such as sodium citrate or sodium phosphate, in an amount of from 0.1 to 1.0 percent by weight; dyestuffs, for example, fluorescein sodium salt, in an amount of about 0.1 to 1.0 percent by weight; care materials, such as plant and vegetable extracts, protein and silk hydro lyzates, lanolin derivative compounds, in an amount of 0.1 to 5 percent by weight; antioxidants, radical trapping agents, anti-flaking active ingredients, in an amount of about 0.01 to 2 percent by weight; luster-imparting ingredients, softening agents, combability improving agents and de-fatting agents.

Suitable thickeners for use in the present invention are Locust Bean Gum, guar-based thickeners, xanthan thickeners (such as xanthan gum sold under the tradename Keltrol^{®} by CPKelco; dehydroxanthan gum sold under the tradename Amaze^{®} XT by National Starch); carageenaan, and Sterculia Urens Gum sold e.g. under the name Karaya Gum.

The compositions may also comprise physiologically compatible silicone derivative compounds, such as volatile or non-volatile silicone oils or high-molecular-weight siloxane polymers in an amount of from 0.05 to 15 percent by weight, preferably from 0.05 to 20 percent by weight, preferably 0.05 to 10 percent by weight. Suitable silicones are e.g. cyclomethicone, dimethicone, dimethiconcopolyol, silicone emulsions, silicone waxes, silicone gums, amodimethicone.

### Product forms

The products of the invention can be used in various product forms such as aerosol mousse, pump mousse, gel, spray-gel, aerosol hairspray, pump spray, hair wax, hair styling cream or hair setting lotion.

One embodiment of the invention is an aerosol-mousse product comprising a composition as described herein wherein the composition additionally comprises at least one propellant and water and wherein the film-forming ingredient is preferably at least one film-forming cationic polymer. The aerosol-mousse product generally comprises a foamable composition in combination with a foaming device. The composition comprises a foam-forming substance, e.g. at least one foam-forming surfactant or at least one foam-forming polymer. A foaming device is a device which effects the forming of a foam or mousse when the composition is dispensed from the container. Suitable devices are commercially available foam-heads.

The amount of propellant may be for example from 1 to 20 or from 2 to 10 weight %. Suitable propellants are for example propane, butane, isobutane or dimethyl ether. The composition is foamed immediately before use and can be applied to wet or dry hair and is left in the hair without rinsing. The film forming ingredients for use in the aerosol-mousse product are preferably selected from the group consisting of polyvinylpyrrolidone, vinylpyrrolidone/vinyl acetate copolymers, chitosan (e.g. in the form of a chitosan salt such as the lactate, formate or pyrrolidone carboxylate), cationic cellulose compounds, copolymers of vinylpyrrolidone, vinylcaprolactam and a basic acrylamide monomer (e.g. dimethyl aminopropylacrylamide), polymers comprising monomeric units of quaternized dialkyl aminoalkylmethacrylate and polymers comprising monomeric units of quaternized vinylimidazolin. Cationic cellulose compounds are for example polyquatemium-4 or polyquatemium-10. Most preferred cationic polymers are selected from the group consisting of copolymers ofN-vinylpyrrolidone, N-vinylimidazol, methacrylamide and quatemized N-vinylimidazol, copolymers of vinyl pyrrolidone and quatemized dimethyl aminoethylmethacrylate and copolymers ofN-vinylpyrrolidone and quatemized N-vinylimidazol and mixtures thereof.

In one embodiment, the product of the invention is in the form of an aerosol-mousse product and comprises a composition comprising
a) from 20 to 95 wt.% of water,
b) from 0,005 to 5 wt.% of spherical shaped polyethylene particles and optionally precipitated silica particles,
c) from 0,1 to 15 wt.% of a film-forming ingredient selected from the group consisting of copolymers ofN-vinylpyrrolidone, N-vinylimidazol, methacrylamide and quatemized N-vinylimidazol and the polymeric salt formed by the reaction of diethyl sulfate and a copolymer of vinyl pyrrolidone and dimethyl aminoethylmethacrylat and mixtures thereof; and
d) from 1 to 20 wt.% of a propellant selected from the group consisting of propane, n-butane and isobutane and mixtures thereof. The aerosol mousse product preferably contains additionally chitosan.

If the hair styling product according to the invention is provided in the form of an aerosol hairspray, the composition is non-foaming and additionally contains from 15 to 85 percent by weight, preferably from 25 to 75 percent by weight, of a propellant, and the composition is filled into a pressurized container having a spray head. Suitable as propellants are lower alkanes, such as n-butane, isobutene and propane, as well as mixtures thereof, as well as dimethyl ethers or fluorohydrocarbons, such as F 152a (1,1-difluoroethane) or F 134 (tetrafluoroethane), as well as propellants which are present at the pressures in question in gaseous forms, such as N₂, N₂O and CO₂, as well as mixtures of the propellants mentioned in the preceding.

If the hair styling product according to the invention is provided in the form of a pump spray, the composition is sprayable and sprayed by means of a suitable, mechanical spraying device. Spray devices of the mechanical type are devices which allow the spraying of a composition without the use of a propellant. Suitable mechanical spray devices are, e.g., spray pumps. Other spray devices are elastic containers provided with a spray valve, into which the cosmetic composition according to the invention is filled under pressure, wherein the elastic container is expanded and wherein the cosmetic composition is continuously discharged if the valve is open, due to the contraction of the elastic container.

If the hair styling product according to the invention is provided in the form of a pump mousse (pump foam), the composition contains at least one conventional foaming substance known in the art for this purpose. The composition is foamed without the aid of propellant gases or chemical propellants and worked into the hair as a foam, and then left in the hair without being rinsed out. A pump mousse product according to the invention has, as an additional component, a device for foaming the composition. Considered as devices for foaming are devices which allow a liquid to be foamed upon dispensing. For example, a commercially available foam pump together with a foaming head can be used as a suitable mechanical foaming device.

If the hair styling product according to the invention is provided in the form of a hair gel or in the form of a spray-gel, it additionally contains at least one gel-forming substance in an amount of preferably from 0.05 to 10, particularly preferably from 0.1 to 2, percent by weight. The viscosity of the gel preferably amounts to from 100 to 50,000 mm²/s, particularly preferably from 1,000 to 15,000 mm²/s, at 25° C., measured as dynamic viscosity using a Bohlin Rheometer CS, measurement body C25 at a shear velocity of 50 s⁻¹. A spray-gel product additionally comprises a mechanical spraying device.

If the hair styling product according to the invention is provided in the form of a hair wax, it additionally contains water-insoluble fatty substances or waxy substances, or substances that provide the composition with a waxy consistency, in an amount of preferably 0.5 to 30 percent by weight. Suitable water-insoluble substances are, for example, emulsifiers having an HLB-value of below 7, silicone oils, silicone waxes, wax materials (e.g., wax alcohols, wax acids, wax esters, as well as, in particular, natural waxes such as beeswax, Carnauba wax, etc.), fatty alcohols, fatty acids, fatty acid esters or high-molecular polyethylene glycols having a molecular weight of from 800 to 20,000, preferably from 2,000 to 10,000, g/mol.

If the hair styling product according to the invention is provided in the form of a hair setting lotion, it is present as a substantially non-viscous or low-viscous solution, dispersion or emulsion, each capable of flowing, with a content of at least 10 percent by weight, preferably 20 to 95 percent by weight, of a cosmetically compatible alcohol. Suitable for use as alcohols are particularly the lower C1 to C4 alcohols usually used for cosmetic purposes, such as ethanol and isopropanol, for example. The viscosity of the setting lotion preferably amounts to less than 100 mm²/s, particularly preferably less than 50 mm²/s, at 25° C., measured as dynamic viscosity using a Bohlin Rheometer CS, measurement body C25 at a shear velocity of 50 s⁻¹.

If the hair styling product according to the invention is present in the form of a hair cream, it is preferably provided in the form of an emulsion and either it contains additional viscosity-providing components in an amount of from 0.1 to 10 percent by weight, or the required viscosity and creamy consistency are built up in the normal way by means of micelle formation, with the help of suitable emulsifiers, fatty acids, fatty alcohols, waxes, etc.

### METHOD OF USE

One embodiment of the invention is a method of treating hair by applying the composition or the aerosol-mousse product as described above to wet or dry hair. Said method preferably comprises the steps of:
a) providing a leave-in hair styling product according to the invention as described above,
b) applying the composition of said leave-in hair styling product to wet hair or to dry hair before, during or after setting the hair into the desired hair style and
c) leaving the composition on the hair without subsequent rinsing.

Such method generally involves application of an effective amount of the product to dry, slightly damp, or wet hair preferably before the hair is arranged to a desired style. The composition is then dried or allowed to dry. By "effective amount" is meant an amount sufficient to provide the hair volume and style benefits desired considering the length and texture of the hair. In general, from about 0.5 g to about 50 g of product will be applied to the hair, depending upon the particular product formulation, length of hair, and type of hair style.

### EXAMPLES

### Example 1: Volume mousse

| | Example 1a | Comparative Example 1a | Example 1b | Comparative Example 1b |
|---|---|---|---|---|
| Sipernat 22 LS | 0,1 | ----- | 0,1 | ----- |
| Microthene FN 510-00 | 0,5 | ------ | 0,5 | ------ |
| Polyquaternium-11 (Gafquat 755 N) | 11,4 | 11,4 | 11,4 | 11,4 |
| Laureth-4 | 0,23 | 0,23 | 0,23 | 0,23 |
| Cetrimoniumchloride (25% solution) | 0,16 | 0,16 | 0,16 | 0,16 |
| Ethanol | 14 | 14 | - | - |
| Water, DI | Add to 100 | Add to 100 | Add to 100 | Add to 100 |

Both formulations have been put in an appropriate volume mousse container, whereby the containers comprised 94 wt% of the formulation and 6 % of propane/butane propellant (4.9 bar).

### Visible volume

To compare visible volume obtainable with the two volume mousses, the mousses have been used as follows.

### Hair swatches:

Hair tresses having a width of 2.5 cm and a length of 22 cm
Mass: 3.0 g ± 0.05g
Characteristics: Cysteic acid: 17.4 - 18.1 µmol /g hair; medullated hair, Ø: 60-80 µm

### Pre treatment (bleaching):

The hair swatches are weighed and then soaked in a mixture of 2.5 g bleaching powder and 7.5 ml Welloxyd per 1 g of hair (in a lab basin). It has to be carried out in an extractor hood. The concentration of hydrogen peroxide in Welloxyd is 9%. The residence time during the bleaching is 30 min. The swatches are turned upside down after each 7.5 min. After bleaching the swatches are rinsed under tap water (6 1 / min, 35°C) for 2 min. The bleaching process is carried out twice in which the 2nd bleaching process follows immediately after the 1^{st}. Before the 2^{nd} bleaching process is started, the hair is dabbed with a napkin. Subsequently the swatches are washed twice with 0.25 ml standard-shampoo (10% Na-laurylether-sulfate, 4% NaCl) per 1 g hair for 1 minute, rinsed for 1 minute. Then the swatches are stored in distilled water for 24h. After that the swatches are rinsed for 2 min under tap water (6 1 / min, 35°C). Finally the swatches are dried at 20°C and 65% r. h. at least over night.

### Treatment with volume mousse of Example I and Comparative Example I (applied as mousse):

3.5 ml mousse is applied with a syringe equally on a swatch with a residual humidity of 50 percent by weight and massaged equally for 1 min. For the volume measurement each swatch is twisted 6 times along the longitudinal axes of the swatch with a constant draw to form a plait. Afterwards the plaits are dried in the drying cabinet (45°C) for 45 min. Then the plaits are stored in the climatic chamber (20°C, 65% r. h.) at least over night.

The definitions of hair volume and body are essentially based on visual perception ('volume') and tactile sensation ('body'). The visually perceptible volume of the swatches is measured by a projection area based on images of a tress photographed from four sides.

The tresses treated as set out below are taken out of the climate chamber and are immediately thereafter suspended from a hook in the measuring chamber (20°C, 65% r. h.). A light box with frosted glass illuminates the swatch from one side and a camera positioned at the opposite side takes the images while the tress is rotated by means of a motor above the hook. During the rotation the camera takes and saves several images to obtain data on the 3-dimensional shape of the tress. With the images a pseudo 3 dimensional volume which envelopes the shape of the tress can be calculated. 60 half-images are taken per rotation, each of which is vertically divided in 40 levels for evaluation. The volume can be determined for each half-image and each level separately; their sum is the total volume. The average of the total volume obtained for 3 different samples is given in Table 1.

**Table 1:**

| | Volume [cm³] |
|---|---|
| Example 1a | 374 ± 50 |
| Comparative Example 1a | 297 ± 20 |
| Example 1b | 310 ± 27 |
| Comparative Example 1b | 258 ± 30 |

Hence, the volume mousse comprising the particles according to the present invention shows significantly higher visible volume.

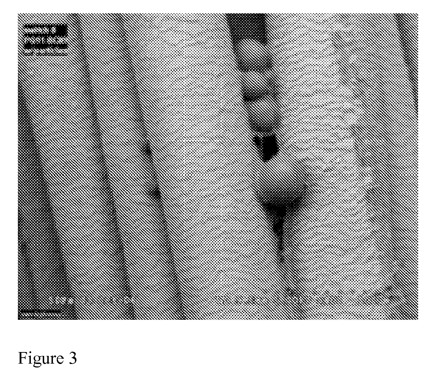

Figure 3 shows a SEM photo of a hair swatch used in the visible volume test and treated with the mousse of Example 1. The SEM used is a Zeiss Ultra 55.

### Combing force

The volume mousses of Example 1 and Comparative Example 1 have further been subjected to a test for combing force.

### Hair swatches:

Hair tresses having a width of 2.5 cm and a length of 17 cm

| | |
|---|---|
| Mass: 2.0 g ± 0.05g | |
| Characteristics: | Cysteic acid: 35.9 µmol /g hair |
| | medullated hair, Ø: 60-80 µm |

### Pre treatment (bleaching):

The hair swatches are weighed and then soaked in a mixture of 2.5 g bleaching powder and 7.5 ml Welloxyd per 1 g of hair (in a lab basin). It has to be carried out in an extractor hood. The concentration of hydrogen peroxide in Welloxyd is 9%. The residence time during the bleaching is 30 min. The swatches are turned upside down after each 7.5 min. After bleaching the swatches are rinsed under tap water (6 1 / min, 35°C) for 2 min. The bleaching process is carried out twice in which the 2nd bleaching process follows immediately after the 1^{st}. Before the 2^{nd} bleaching process is started, the hair is dabbed with a napkin. Subsequently the swatches are washed twice with 0.25 ml standard-shampoo (10% Na-laurylether-sulfate, 4% NaCl) per 1 g hair for 1 minute, rinsed for 1 minute. Then the swatches are stored in distilled water for 24h. After that the swatches are rinsed for 2 min under tap water (6 1 / min, 35°C). Finally the swatches are dried at 20°C and 65% r. h. at least over night.

### Treatment with volume mousse of Example 1 and Comparative Example 1 (applied as mousse):

3.5 ml mousse is applied with a syringe equally on a swatch with a residual humidity of 50 percent by weight and massaged for 1 min. Afterwards the swatches are combed.

Various external influences, such as certain cosmetic treatments (bleaching, coloring, permanent waving), exposure to the weather, frequent combing and brushing affect the combing ease due to a degradation of the hair cuticle. Thus the combing ease of hair is a major parameter for describing the quality of hair on one side and or the effectiveness of conditioning treatments on the other side.

The principle of this method for determination of combability properties consists in measuring the force to pull a comb through a hair tress under definite conditions.

For this experiment an automated device is used which takes the tresses to be tested from a storage chamber (20°C, 65% r. h.) and fixes them to a force meter (HBM, type Z6FD1) (20°C, 65% r. h.). The tresses are combed automatically, one by one, at constant speed. For each combing cycle the combing force is recorded as a function of the distance combed through (length of the tress). For comparison purposes 20 combing strokes per swatch on 3 swatches (i.e. 3 swatches for Example 1 and 3 swatches for Comparative Example 1) are averaged. The results on combing force are given in Table 2.

**Table 2:**

| | Combing force [N] |
|---|---|
| Example 1 | 0,034 ± 0,007 |
| Comparative Example 1 | 0,043 ± 0,010 |

Hence, the volume mousse comprising the particles according to the present invention shows significantly lower combing force, i.e. the hair is easier to comb.

Further examples for the leave-in compositions according to the present invention are given in the following formulations.

### Example 2: Volume Spray Gels

| | Ex. 2a | Ex. 2b | Ex. 2c | Ex. 2d | Ex. 2e |
|---|---|---|---|---|---|
| Sipernat 22 LS | 0.1 | ----- | ----- | 0.1 | 0.1 |
| Microthene FN 510-00 | 0.5 | 0.6 | 0.8 | 0.5 | 0.5 |
| Aculyn 28 | 1.4 | 1.4 | 1.4 | - | - |
| HydroxypropylGuar (Jaguar HP-60) | - | - | - | - | 0.5 |
| AMP 95% | 0.1 | 0.1 | 0.1 | 0.1 | 0.1 |
| vinylpyrrolidone/vinylacet ate copolymer (Luviskol VA 64) | 3.0 | 3.0 | 3.0 | 3.0 | 3.0 |
| polyvinylpyrrolidone (Luviskol K30) | - | - | - | 2 | 2 |
| low mw chitosan (mw about 20000-100000) | - | - | - | 0.55 | 0.55 |
| high mw chitosan (mw about 300000 - 700000) | - | - | - | 0.15 | 0.15 |
| formic acid (85%) | - | - | - | 0.5 | 0.75 |
| Castor Oil PEG-40 H, (90%) | 0.5 | 0.5 | 0.5 | 0.4 | 0.4 |
| PEG-90 hydrogenated castor oil | - | - | - | 0.2 | 0.2 |
| polysorbate-20 | - | - | - | 0.4 | 0.4 |
| polysorbate-80 | - | - | - | 0.2 | 0.2 |
| Disodium EDTA | - | - | - | 0.13 | 0.13 |
| Perfume | 0.2 | 0.2 | 0.2 | 0.1 | 0.1 |
| Euxyl PE 9010 | 0.7 | 0.7 | 0.7 | 0.4 | 0.4 |
| Nipaguard DMDMH | 0.4 | 0.4 | 0.4 | - | - |
| PHB-methylester | - | - | - | 0.2 | 0.2 |
| Water, DI | Add to 100 | Add to 100 | Add to 100 | Add to 100 | Add to 100 |

The composition is placed in a container which is equipped with a suitable mechanical pump spray device.

### Example 3: Styling Gel

| | **Example 3** |
|---|---|
| Sipemat 22 LS | 0.005 |
| Microthene FN 510-00 | 0.005 |
| Stabilize 06 | 1 |
| SODIUM CHLORIDE | 0.25 |
| PVP/VA-W-735 | 6.00 |
| SOLULAN L 575 | 2.0 |
| SURFACTANT 193 | 2.0 |
| Polyox WsR-301 | 0.09 |
| Castor Oil PEG-40 H, (90%) | 1.2 |
| Perfume | 0.2 |
| Ethanol | 1.5 |
| Preservative | 0.6 |
| Water, DI | Add to 100 |

### Example 4: Volume Styling Gels

| | **Example 4a** | **Example 4b** | **Example 4c** |
|---|---|---|---|
| Sipernat 22 LS | 0.1 | ----- | ----- |
| Microthene FN 510-00 | 0.5 | 0.6 | 0.8 |
| Aculyn 28 | 2.3 | 2.3 | 2.3 |
| Polyquaternium 4 | 1.2 | 1.2 | 1.2 |
| cellulose | 0.3 | 0.3 | 0.3 |
| Perfume | 0.3 | 0.3 | 0.3 |
| Rexat | 0.1 | 0.1 | 0.1 |
| Nipaguard DMDMH | 0.3 | 0.3 | 0.3 |
| Water, DI | Add to 100 | Add to 100 | Add to 100 |

### Example 5: Further gels (tube gels)

| | **Example 5a** | **Example 5b** | **Example 5c** |
|---|---|---|---|
| Sipemat 22 LS | 0.1 | ----- | ----- |
| Microthene FN 510-00 | 0.5 | 0.6 | 1.0 |
| Polyvinylpyrrolidone K90 | 2.8 | 2.8 | 2.8 |
| Polyacrylates | 0.7 | 0.7 | 0.7 |
| Aminopropanol | 0.5 | 0.5 | 0.5 |
| Perfume | 0.2 | 0.2 | 0.2 |
| Rexat | 0.1 | 0.1 | 0.1 |
| Nipaguard DMDMH | 0.6 | 0.6 | 0.6 |
| Propylenglycol 1 | 3.0 | 3.0 | 3.0 |
| Glycerine | 4.0 | 4.0 | 4.0 |
| Water, DI | Add to 100 | Add to 100 | Add to 100 |

### Example 6: Nonaerosol Volume pump sprays (setting lotions)

| | **Example 6a** | **Example 6b** | **Example 6c** |
|---|---|---|---|
| Sipernat 22 LS | 0.1 | ----- | ----- |
| Microthene FN 510-00 | 0.5 | 0.6 | 0.8 |
| Vinylpyrrolidon/Vinylacet at Copolymer (Luviskol^{®} VA 55E) | 6.0 | 6.0 | 6.0 |
| Parfume | 0.3 | 0.3 | 0.3 |
| Ethanol 96 % | 40.0 | 40.0 | 40.0 |
| Water, DI | Ad to 100 | Ad to 100 | Ad to 100 |

The composition is placed in a container which is equipped with a suitable mechanical pump spray device.

### Example 7 Volume mousse

| | Example 7a | Example 7b | Example 7c | Example 7d |
|---|---|---|---|---|
| Sipernat 22 LS | 0.1 | 0.1 | 0.1 | 0.1 |
| Microthene FN 510-00 | 0.5 | 0.5 | 0.5 | 0.5 |
| Luviquat Supreme (20% solution of Polyquaternium-68) | 6.4 | 7.7 | - | - |
| Gafquat 755 N (20% solution of Polyquaternium-11) | - | - | 4.1 | 4.9 |
| Luviquat Excellence (40% solution of Polyquaternium-11) | 0.6 | 0.8 | 0.4 | 0.45 |
| low mw chitosan (mw about 20000-100000) | 0.17 | 0.2 | 0.2 | 0.24 |
| high mw chitosan (mw about 300000 - 700000) | 0.12 | 0.14 | 0.14 | 0.16 |
| formic acid (85%) | 0.07 | 0.09 | 0.10 | 0.13 |
| Luviskol K 85 CQ (20% solution of polyvinylpyrrolidone) | 2.9 | 3.5 | 3.5 | 4.2 |
| vinylpyrrolidone/vinylacetate copolymer | 0.42 | 0.5 | 0.5 | 0.6 |
| Laureth-4 | 0.11 | 0.11 | 0.15 | 0.15 |
| Cetrimoniumchloride (25% solution) | 0.4 | 0.4 | 0.35 | 0.4 |
| PHB-methylester | 0.1 | 0.1 | 0.1 | 0.1 |
| phenoxetol | 0.5 | 0.5 | 0.5 | 0.5 |
| sodium EDTA | 0.13 | 0.13 | 0.14 | 0.14 |
| perfume | 0.2 | 0.2 | 0.2 | 0.2 |
| Water, DI | ad 100 | ad 100 | ad 100 | ad 100 |

The formulations have been put in an appropriate pressure resistant volume mousse container, whereby the containers comprised 94 wt% of the formulation and 6 % of propane/butane propellant (4.8 bar).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A leave-in hair styling product wherein the product is in the form of an aerosol mousse, pump mousse, gel, spray-gel, aerosol hairspray, pump spray, hair wax, hair styling cream or hair setting lotion and the product comprises a composition, said composition comprising
a) a liquid carrier,
b) at least one hair volume increasing, solid particulate material selected from the group consisting of
(i) solid particles of spherical, ellipsoid or oval shape having a mean particle size of from 5 µm to 80 µm, a water content of below 5 wt.% and the solid particals do not melt at temperatures below 90°C, and
(ii) a mixture of at least one first solid material and at least one second solid material wherein said first solid material is at least one irregularly shaped particle and said second solid material is at least one spherical particle, wherein said irregularly shaped particles have a particle size of at least 0,01 micron, said spherical shaped particles have a particle size of at least 0,01 micron and the median particle size of said spherical particle is greater than the median particle size of said irregular shaped particle,
c) at least one film-forming ingredient.

2. The product of claim 1, wherein the spherical, ellipsoid or oval shaped particles have a density of less than 2.1 g/cm³ and comprise at least 80% of a polyolefin.

3. The product of claim 1 or 2, wherein the liquid carrier is selected from the group consisting of water, ethanol, isopropanol and mixtures thereof.

4. The product of any of the preceding claims, wherein the film-forming ingredient is selected from the group consisting of cationic polymers and nonionic vinyllactam polymers and mixtures thereof.

5. The product of claim 4, wherein the cationic polymers are selected from the group consisting of polymers comprising monomeric units of quaternized dialkyl aminoalkylmethacrylate and polymers comprising monomeric units of vinylimidazolin and wherein the nonic vinyllactam polymer is selected from polyvinylpyrrolidone and copolymers of vinylpyrrolidone and vinylacetate and mixtures thereof.

6. The product of claim 5, wherein the cationic polymer is selected from the group consisting of copolymers ofN-vinylpyrrolidone, N-vinylimidazol, methacrylamide and quatemized N-vinylimidazol, copolymers of vinyl pyrrolidone and quatemized dimethyl aminoethylmethacrylate and copolymers ofN-vinylpyrrolidone and quatemized N-vinylimidazol and mixtures thereof.

7. The product of any of the preceding claims, wherein the total amount of liquid carrier is from 20 to about 95 wt.% (preferably from 55 to 85 wt.%), the total amount of particles is from 0,005 to 5 wt.% and the total amount of film-forming polymers is from 0,1 to 15 wt.% (preferably from 0,5 to 10 wt.%).

8. The product of any of the preceding claims, wherein the product is in the form of an aerosol-mousse product comprising a composition of any of the preceding claims and the composition additionally comprises at least one propellant.

9. The product of any of the preceding claims, wherein the product is in the form of an aerosol-mousse product said composition comprising
a) from 20 to 95 wt.% of water,
b) from 0,005 to 5 wt.% of spherical shaped polyethylene particles and optionally precipitated silica particles,
c) from 0,1 to 15 wt.% of a film-forming ingredient selected from the group consisting of copolymers ofN-vinylpyrrolidone, N-vinylimidazol, methacrylamide and quatemized N-vinylimidazol and the polymeric salt formed by the reaction of diethyl sulfate and a copolymer of vinyl pyrrolidone and dimethyl aminoethylmethacrylat and mixtures thereof; and
d) from 1 to 20 wt.% of a propellant selected from the group consisting of propane, n-butane and isobutane and mixtures thereof.

10. The product according to claim 8 or 9, additionally containing chitosan.

11. A method of treating hair comprising the steps of
- providing a leave-in hair styling product according to any of the product claims,
- applying the composition of said leave-in hair styling product to wet hair or to dry hair, and
- leaving the composition on the hair without subsequent rinsing.

12. Use of the leave-in hair styling product of any of the preceding product claims for improving the volume of a hair-do.
